(19) Europäisches Patentamt
European Patent Office
Office européen des brevets

(11) EP 0 545 135 B1

# (12) EUROPÄISCHE PATENTSCHRIFT

(45) Veröffentlichungstag und Bekanntmachung des Hinweises auf die Patenterteilung:
**17.06.1998 Patentblatt 1998/25**

(51) Int. Cl.[6]: **C01B 13/36**, C01F 7/34, C01G 23/053, C01G 25/02

(21) Anmeldenummer: **92119595.4**

(22) Anmeldetag: **17.11.1992**

## (54) Herstellung von Metalloxidsolen durch Elektrolyse

Production of sols of metal oxides by electrolysis

Fabrication d'un sol d'oxyde métallique par électrolyse

(84) Benannte Vertragsstaaten:
**DE ES FR GB IT**

(30) Priorität: **30.11.1991 DE 4139579**

(43) Veröffentlichungstag der Anmeldung:
**09.06.1993 Patentblatt 1993/23**

(73) Patentinhaber: **MERCK PATENT GmbH**
**D-64271 Darmstadt (DE)**

(72) Erfinder:
- **Parusel, Manfred**
  **W-6115 Münster (DE)**
- **Ambrosius, Klaus, Dr.**
  **W-6110 Dieburg (DE)**
- **Franz, Klaus-Dieter, Dr.**
  **W-6223 Kelkheim (DE)**
- **Hechler, Wolfgang**
  **W-6147 Lautertal (DE)**
- **Schraml-Marth, Matthias, Dr.**
  **W-6144 Zwingenberg (DE)**

(56) Entgegenhaltungen:
- **CHEMICAL ABSTRACTS, vol. 112, no. 24, 11. Juni 1990, Columbus, Ohio, US; abstract no. 224067, SHARYGIN L.M. ET AL. 'Colloid chemical transformations of titanium (IV) during electrolysis of an aqueous solution'**
- **CHEMICAL ABSTRACTS, vol. 102, no. 18, 6. Mai 1985, Columbus, Ohio, US; abstract no. 156804, SHARYGIN L.M. ET AL. 'Formation of a zirconium hydroxide sol in electrolysis of dichlorooxozirconium solution'**
- **IDEM**
- **CHEMICAL ABSTRACTS, vol. 101, no. 8, 20. August 1984, Columbus, Ohio, US; abstract no. 60740, SHARYGIN L.M. ET AL. 'Study of the formation of a hydrated titanium dioxide sol by Raman spectroscopy'**
- **IDEM**
- **CHEMICAL ABSTRACTS, vol. 114, no. 24, 17. Juni 1991, Columbus, Ohio, US; abstract no. 235720, SHARYGIN L.M. ET AL. 'Colloid-chemical transformations during electrolysis of a two-component aqueous tin (IV) chloride titanium (IV) chloride solution'**
- **IDEM**
- **CHEMICAL ABSTRACTS, vol. 95, no. 14, 5. Oktober 1981, Columbus, Ohio, US; abstract no. 122966, SHARYGIN L.M. ET AL. 'Preparation of aqueous sols of hydrated oxides of zirconium, titanium and tin by the electrolysis of their chlorides'**
- **DATABASE WPIL Week 8313, Derwent Publications Ltd., London, GB; AN 83-31757K/13**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist. (Art. 99(1) Europäisches Patentübereinkommen).



Printed by Xerox (UK) Business Services
2.16.3/3.4

## Beschreibung

Die Erfindung betrifft die Herstellung von ein- oder mehrkomponentigen Metalloxidsolen aus wäßrigen Metallsalzlösungen bzw. einer Lösung eines Metallsalzgemisches durch Elektrolyse.

Verfahren für die Herstellung von Metalloxidsolen sind in der Literatur vielfach beschrieben. Die Herstellung der Sole erfolgt zumeist, indem eine wäßrige Lösung eines Metallsalzes hergestellt wird, welche dann z.B. durch Hydrolyse, die durch Erhitzen erwirkt werden kann, und/oder durch saure Peptisierung und/oder durch Zugabe einer Base, in den Solzustand überführt wird. Nachteilig bei diesen Verfahren ist, daß häufig anstelle der Solbildung Ausfällungen stattfinden, was insbesondere bei der Verwendung von teuren Metallsalzen, z.B. Organometallkomplexen von großem Nachteil ist. Somit ist man häufig bei der Solherstellung auf kleine Metallsalzkonzentrationen beschränkt.

Ein verbessertes Verfahren für die Herstellung der Kolloide arbeitet mit Ionenaustauschern. Von Nachteil ist allerdings bei diesem Verfahren die begrenzte Austauschkapazität sowie das geringe Reaktorvolumen. Nach Beendigung jedes Prozesses muß der Ionenaustauscher erneuert und regeneriert werden, so daß der Prozeß nur batchweise ablaufen kann.

Metallsole können auch durch Elektrodialyse erhalten werden, wobei jedoch Solbildung und Ausfällungen häufig miteinander konkurrieren.

Elektrolyseverfahren zur Herstellung kolloidaler Titan- und Zinnsalzlösungen sind bereits aus der SU 706468 und SU 929741 A sowie aus der Kolloidn. Zh. 43 (4), 192-5 und 812-16, 42(1), 188-191, 46(3), 607-609, 47(1), 120-125 und 53(1), 178-181 bekannt.

Die Herstellung eines Titanoxidsols durch Elektrolyse einer $TiCl_4$-Lösung gelingt allerdings nur in Gegenwart eines stabilisierenden Additivs wie z.B. $ZrCl_4$. Bei der Elektrolyse der reinen Metallsalzlösung wurden verstärkt Zersetzungen bzw. Ausfällungen bei der Solbildung beobachtet. Bei den in der Literatur beschriebenen Elektrolyseverfahren werden dreikammerige Elektrolyseeinheiten benutzt, was kompliziert ist und die Verwendung von Ionenaustauschmembranen erfordert.

Mehrkomponentige Metalloxidsole können durch Hydrolyse von Metallalkoxidgemischen in hoher Reinheit und bei niedrigen Temperaturen hergestellt werden. Nachteil dieser Methode ist der sehr hohe Preis der Metallalkoxide.

Es bestand somit ein Bedürfnis, ein einfaches und wenig aufwendiges Verfahren zu finden, in dem stabile ein- oder mehrkomponentige Metalloxidsole ohne stabilisierenden Additivzusatz in hohen Ausbeuten hergestellt werden können.

Überraschenderweise wurde nun ein Verfahren zur Herstellung von ein- oder mehrkomponentigen Metalloxidsolen gefunden, in dem die bei herkömmlichen Verfahren genannten Nachteile nicht oder nur in geringem Umfang auftreten.

Gegenstand der Erfindung ist somit ein Verfahren zur Herstellung von ein- oder mehrkomponentigen Metalloxidsolen, dadurch gekennzeichnet, daß man eine wäßrige Metallsalzlösung bzw. die Lösung eines Metallsalzgemisches bei 0-15°C durch direkte Elektrolyse ohne stabilisierenden Additivzusatz hydrolysiert, wobei die Metallsalzlösung bzw. die Lösung eines Metallsalzgemisches kontinuierlich durch die Elektrolysezelle gepumpt wird. Als Metallsalze werden insbesondere Titan-, Aluminium-, Zirkonium-, Hafnium-, Niob-, Tantal-, Yttrium-, Lanthan-, Actinid- und/oder Lanthanidverbindungen verwendet.

Die Herstellung der ein- oder mehrkomponentigen Metalloxidsole erfolgt auf einfache Weise, indem man ein Metallsalz bzw. ein Metallsalzgemisch in Wasser löst und mehrere Stunden elektrolysiert, wobei die wäßrige Metallsalzlösung in einem Kreislauf kontinuierlich durch die Elektrolysezelle gepumpt wird. Der Solbildungsprozeß findet bereits bei niedrigen Temperaturen statt. Der Temperaturbereich betragt 0-15°C.

Bei der Herstellung der erfindungsgemäßen ein- oder mehrkomponentigen Metalloxidsole treten die im Stand der Technik genannten Nachteile nicht oder nur bedingt auf, da durch die Wahl der Elektrolyseapparatur

a) Inhomogenitäten in der Lösung durch Umpumpen, Rühren bzw. Durchleiten von Luft vermieden werden, und

b) durch Kühlung ein Ansteigen der Temperatur verhindert wird.

Das Elektrodenmaterial spielt bei der Solherstellung ebenfalls eine Rolle. Geeignete Elektrodenmaterialien sind insbesondere Titan-Metall-Gitterelektroden beschichtet mit Ruthenium- bzw. Iridiumoxid.

Alle bekannten Metallsalze, die an der Kathode praktisch nicht reduzierbar sind, d.h. unter den Reaktionsbedingungen ein positiveres Elektrodenpotential aufweisen als das System $H_2/H^+$ an dieser Elektrode, sind für das Verfahren geeignet. Vorzugsweise werden die Oxidhalogenide und Halogenide der Metalle eingesetzt, insbesondere die Chloride. Lösungen von Metallsalzgemischen bestehend aus zwei oder mehreren verschiedenen Metallsalzen können ebenfalls nach dem erfindungsgemäßen Verfahren in homogen durchmischte Metalloxidsole überführt werden. Vorzugsweise werden wäßrige Lösungen enthaltend maximal drei verschiedene Metallsalze elektrolysiert.

Für die Elektrolyse von Mischungen wäßriger Metallsalzlösungen sind insbesondere Zirkonium-Titan-, Zirkonium-Aluminium-, Zirkonium-Cer-, Zirkonium-Yttrium-, Zirkonium-Cer-Lanthan- und Titan-Aluminium-Salzlösungen geeignet.

Mehrkomponentige Metalloxidsole können auch durch Mischen von zwei oder mehr separat hergestellter einkomponentiger Metalloxidsole hergestellt werden. Letzteres ist allerdings ein sehr aufwendiges und kostenintensives Verfahren.

Bei der Herstellung mehrkomponentiger Metalloxidsole durch Elektrolyse von zwei oder mehr verschiedenen Metallsalzlösungen liegen in den ca. 20 nm großen Solpartikeln bereits heteropolare, d.h. z.B.: Zr-O-Ti-Bindungen vor, was bei Mischungen bestehend aus verschiedenen einkomponentigen Metalloxidsolen mit z.B. Zr-O-Zr- und Ti-O-Ti-Bindungen nicht der Fall ist.

Bei dem erfindungsgemäßen Verfahren gibt es keine Konzentrationsbeschränkungen hinsichtlich der einzusetzenden Metallsalzlösung. Üblicherweise wird im Konzentrationsbereich zwischen 0,5-40 Gew.% bezogen auf das entstehende Metalloxid gearbeitet.

Gegenstand der Erfindung ist somit auch, daß die Metallsalzlösung bzw. eine Lösung eines Metallsalzgemisches in einer Konzentration von 0,5-40 Gew.% bezogen auf das entstehende Metalloxid eingesetzt wird.

Stromstärke, Stromspannung und Elektrolysedauer können variieren. Die angelegte Spannung pro Elektrolysezelle liegt zwischen 2 V (Zersetzungsspannung des Wassers) und 20 V, vorzugsweise zwischen 5 und 10 V. Die resultierende Stromstärke liegt im Bereich von 0,01 bis 0,5 A/cm$^2$ und sinkt kontinuierlich während des Elektrolyseprozesses.

Gegenstand der Erfindung ist somit auch, daß bei der Elektrolyse mit Spannungen zwischen 2-20 V und einer Stromstärke zwischen 0,01-0,5 A/cm$^2$ gearbeitet wird.

Die Elektrolysedauer ist abhängig von dem Verhältnis aus Metallsalzmenge und Stromstärke, z.B. erfordert die Umwandlung von 1 l $TiCl_4$-Lösung mit ca. 350 g $TiCl_4$/l bei je 100 cm$^2$ Anoden bzw. Kathodenfläche (15 A Anfangsstärke bei konstant 5 V Spannung) 25-30 h.

Das erfindungsgemäße Verfahren verläuft gemäß folgender Gleichungen:

$$MeX_n + H_2O \xrightleftharpoons{\text{Hydrolyse}} Me(OH)X_{n-1} + HX$$

$$2\,HX \xrightarrow{\text{Elektrolyse}} H_2\uparrow + X_2\uparrow$$

Me = Metall(e)
X = gängige Anionen.

Die erfindungsgemäß erhaltenen Metalloxidsole zeichnen sich durch ihre hohe Transparenz und ihre Teilchengröße aus. Die Solteilchen weisen eine mittlere Teilchengröße zwischen 5 bis 1000 nm auf, insbesondere zwischen 10 bis 100 nm.

Die Konzentrationen der Sole sind abhängig von der Einsatzkonzentration der Salzlösung und liegen bei 0,5-40 % Metalloxid.

Überraschenderweise zeigte sich, daß bei dem erfindungsgemäßen Verfahren keine Stabilisierung durch Zusätze erforderlich ist; dies ist vermutlich zurückzuführen auf das weitgehende Fehlen störender Einflüsse wie z.B. zusätzliche Membranen, Konzentrationsunterschiede und hohe Temperaturen.

Die Metalloxidsole sind wegen ihrer hohen Transparenz und variablen Konzentration daher insbesondere, soweit zugelassen, für kosmetische Zubereitungen und in der Keramik als Lüster und Dekorativ bestens geeignet. Die erfindungsgemäß hergestellten $TiO_2$-Sole dienen insbesondere in der Kosmetik als UV-Schutz. In der Keramik werden Titandioxid-Sole eingesetzt, da daraus hergestellte Titandioxidschichten aufgrund ihres hohen Brechungsindexes besonders brillante und ästhetisch eindrucksvolle Interferenzfarben aufzeigen. Die Anwendung von Solen in der Keramik ist z.B. in DE 41 05 235 und in der Kosmetik z.B. in DE 41 19 719 beschrieben.

Mehrkomponentige Metalloxidsole lassen sich problemlos durch Verfahren wie Sprüh-, Gefrier- oder Mikrowellentrocknung in amorphe Pulver überführen, aus denen durch Kalzination bei geeigneten Temperaturen polykristalline Mischoxidpulver variabler Zusammensetzung gewonnen werden können.

Derartige Mischmetalloxide werden vorzugsweise als Katalysatoren, als Trägermaterialien für katalytisch aktive Substanzen, in der Keramik (z.B. Zirkontitanat ($ZrTiO_4$), Aluminiumtitanat ($AlTiO_5$), Cer bzw. Yttrium stabilisiertes $ZrO_2$) und der Chromatographie eingesetzt.

Gegenstand der Erfindung sind somit auch Zubereitungen, die die erfindungsgemäß hergestellten ein- oder mehrkomponentigen Metalloxidsole enthalten.

Die nachfolgenden Beispiele sollen die Erfindung erläutern, ohne sie jedoch zu begrenzen:

Beispiel 1

In eine Glaskolben mit Doppelmantel, versehen mit einem Thermometer und einer pH-Elektrode, werden 1 l $TiCl_4$-Lösung (290 g $TiCl_4$ gelöst in 1 l Wasser) auf 10 °C abgekühlt. Diese Lösung wird kontinuierlich im Kreislauf durch eine Elektrolysezelle gepumpt. Nach Anlegen einer Spannung von 5 V und einer Stromstärke von 15 A/dm$^2$ wird 30 h elektrolysiert, wobei die Stromstärke auf 1 A/dm$^2$ sinkt. Der pH-Wert des erhaltenen Titanoxidsoles beträgt 1,8. Bei dem Sol handelt es sich um eine wasserklare, viskose Flüssigkeit.

Beispiel 2

In der in Beispiel 1 beschriebenen Apparatur werden 1 l $ZrOCl_2$-Lösung (260 g $ZrO_2$ x 8 $H_2O$ gelöst in 1 l Wasser) auf 5 °C abgekühlt. Nach Anlegen einer Spannung von 5 V und einer Stromstärke von 6,5 A/dm$^2$ wird 20 h elektrolysiert. Die Stromdichte beträgt am Schluß 0,2 A/dm$^2$. Der pH-Wert des erhaltenen farblosen, klaren Sols beträgt 2,6.

Beispiel 3

Analog Beispiel 1 werden eine $ZrOCl_2$-$TiCl_4$-Lösung (158,2 g $ZrOCl_2 \cdot 8 H_2O$ und 262 ml $TiCl_4$-Lösung (357 g $TiCl_4$ gelöst in 1 l Wasser) in 1 l Wasser) bei 5 °C, einer Anfangsspannung von 5 V und einer Stromstärke von 13,7 A/dm$^2$ 18 h elektrolysiert, wobei die Stromstärke während des Solbildungsprozesses auf 1 A/dm$^2$ sinkt.

Das erhaltene wasserklare Zirkontitanat-Sol kann durch die bekannten Trocknungsverfahren in ein weißes rieselfähiges Pulver umgewandelt werden.

Vergleichsbeispiel

Äquimolare Mengen der in Beispiel 1 und Beispiel 2 separat hergestellten Titanoxid- und Zirkonoxid-Sole werden gemischt und die mechanische Mischung wird anschließend getrocknet.

Analog Beispiel 3 bildet sich ab 400 °C kristallines Zirkontitanat.

## Patentansprüche

1. Verfahren zur Herstellung von ein- oder mehrkomponentigen Metalloxidsolen, dadurch gekennzeichnet, daß man eine wäßrige Metallsalzlösung bzw. eine Lösung eines Metallsalzgemisches bei 0-15°C durch direkte Elektrolyse ohne stabilisierenden Additivzusatz hydrolysiert, wobei die Metallsalzlösung bzw. die Lösung eines Metallsalzgemisches kontinuierlich durch die Elektrolysezelle gepumpt wird.

2. Verfahren nach Anspruch 1, dadurch gekennzeichnet, daß es sich bei den Metallsalzen um Aluminium-, Titan-, Zirkonium-, Hafnium-, Niob-, Tantal-, Yttrium-, Lanthan-, Actinid- und Lanthanidverbindungen handelt.

3. Verfahren nach Anspruch 1, dadurch gekennzeichnet, daß bei der Elektrolyse eine Spannung zwischen 2-20 V und eine Stromstärke zwischen 0,01-0,5 A/cm$^2$ angelegt wird.

4. Verfahren nach Anspruch 1, dadurch gekennzeichnet, daß die Metallsalzlösung bzw. eine Lösung eines Metallsalzgemisches in einer Konzentration von 0,5-40 Gew.% bezogen auf das entstehende Metalloxid bzw. Metalloxidgemisch eingesetzt wird.

## Claims

1. Process for preparing one-component or multicomponent metal oxide sols, characterized in that an aqueous metal salt solution or a solution of a metal salt mixture is hydrolysed at 0-15°C by direct electrolysis without addition of a stabilizing additive, where the metal salt solution or the solution of a metal salt mixture is pumped continuously through the electrolysis cell.

2. Process according to Claim 1, characterized in that the metal salts are aluminium, titanium, zirconium, hafnium, niobium, tantalum, yttrium, lanthanum, actinide and lanthanide compounds.

3. Process according to Claim 1, characterized in that a potential of 2-20 V and a current density of 0.01-0.5 A/cm$^2$ are applied during electrolysis.

4. Process according to Claim 1, characterized in that the metal salt solution or solution of a metal salt mixture used has a concentration of 0.5-40% by weight, based on the metal oxide or metal oxide mixture formed.

**Revendications**

1. Procédé de préparation de sols d'oxydes métalliques à un ou plusieurs composants, caractérisé en ce qu'on hydrolyse une solution aqueuse de sels métalliques ou une solution d'un mélange de sels métalliques à une température de 0-15°C par électrolyse directe sans addition d'additifs stabilisants, la solution de sels métalliques ou la solution d'un mélange de sels métalliques étant pompée en continu à travers la cellule d'électrolyse.

2. Procédé selon la revendication 1, caractérisé en ce que, pour ce qui concerne les sels métalliques, il s'agit de composés de l'aluminium, du titane, du zirconium, du hafnium, du niobium, du tantale, de l'yttrium, du lanthane, des actinides et des lanthanides.

3. Procédé selon la revendication 1, caractérisé en ce qu'on applique lors de l'électrolyse une tension comprise entre 2-20 V et une densité de courant comprise entre 0,01-0,5 A/cm$^2$.

4. Procédé selon la revendication 1, caractérisé en ce que la solution de sels métalliques ou une solution d'un mélange de sels métalliques est utilisée à une concentration de 0,5-40 % en poids par rapport à l'oxyde métallique ou au mélange d'oxydes métalliques formé.